# EUROPÄISCHE PATENTANMELDUNG

(11) **EP 3 025 789 A1**
(43) Veröffentlichungstag der Anmeldung: **01.06.2016**
(21) Anmeldenummer: 15192076.6
(22) Anmeldetag: 29.10.2015
(51) Int. Cl.: B05C 17/01, A61B 17/56, A61F 2/46

(54) **AUSTRAGSVORRICHTUNG FÜR ZEMENTKARTUSCHEN MIT ABROLLENDEN KLEMMKÖRPERN**

(30) Priorität: 25.11.2014 DE 102014117224
(71) Anmelder: Heraeus Medical GmbH, 61273 Wehrheim (DE)
(72) Erfinder: Greiner, Clemens, 30826 Garbsen (DE); Dr. Vogt, Sebastian, 99092 Erfurt (DE)
(74) Vertreter: Panten, Kirsten

(57) **Zusammenfassung**

Die Erfindung betrifft eine Austragsvorrichtung (1) für Zementkartuschen für Polymethylmethacrylat-Knochenzemente aufweisend mindestens einen manuell zu betätigenden Kipphebel (4), einen Adapter (44) für eine Zementkartusche, eine in Richtung des Adapters (44) vortreibbare Klemmstange (2), zumindest einen Vortriebskörper (24) angeordnet ist, der in axialer Richtung bezüglich der Achse der Klemmstange (2) verschiebbar ist, wobei mindestens ein kugel- oder walzenförmiger Klemmkörper (26) in einer Kavität des zumindest einen Vortriebskörpers (24) angeordnet ist, wobei ein elastisches Element (28, 52) den mindestens einen Klemmkörper (26) des zumindest einen Vortriebskörpers (24) aus Richtung des Adapters (44) an eine geneigte Fläche oder Kante der Kavität des zumindest einen Vortriebskörpers (24) drückt, wobei ein Federelement (40) entgegengesetzt zur Vortriebsrichtung der Klemmstange (2) auf den zumindest einen Vortriebskörper (24) drückt, zumindest ein Sicherungselement (34) an der Klemmstange (2) anliegt, wobei mindestens ein kugel- oder walzenförmiger Klemmkörper (36) in einer Kavität des zumindest einen Sicherungselements (34) angeordnet ist, wobei ein elastisches Element (38, 50) den mindestens einen Klemmkörper (36) des zumindest einen Sicherungselements (34) aus Richtung des Adapters (44) an eine geneigte Fläche oder Kante der Kavität des zumindest einen Sicherungselements (34) drückt.

Die Erfindung betrifft auch ein Verfahren zum Vortreiben einer Klemmstange (2) mit einem Kipphebel (4) bei dem durch manuelle Krafteinwirkung auf den Kipphebel (4) zumindest ein an der Klemmstange (2) anliegender Vortriebskörper (24) in einer ersten axialen Richtung der Klemmstange (2) bewegt wird.

## Beschreibung

Die Erfindung betrifft eine Austragsvorrichtung für Zementkartuschen für Polymethylmethacrylat-Knochenzemente aufweisend mindestens einen manuell zu betätigenden Kipphebel und einen Adapter für eine Zementkartusche.

Gegenstand der Erfindung ist somit eine manuell angetriebene Vorrichtung zum Austrag von Polymethylmethacrylat-Knochenzementteig aus Kartuschen von Vakuumzementiersystemen. Die Vorrichtung ist zum nur einmaligen Gebrauch bestimmt.

Gelenkendoprothesen werden in der Orthopädie und Unfallchirurgie im breiten Umfang zum Ersatz von menschlichen Gelenken verwendet, wenn diese durch Krankheiten, Unfall oder durch Verschleiß geschädigt sind. Die dauerhafte mechanische Fixierung von Gelenkendoprothesen erfolgt dabei durch mechanische Klemmung (press-fit) oder durch Zementierung mit Polymethylmethacrylat-Knochenzementen (PMMA-Knochenzementen).

PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, die auf Basis von Methylmethacrylat und Comonomeren, wie Styren, Methylacrylat oder ähnlichen Monomeren durch Polymerisation, vorzugsweise Suspensionspolymerisation, hergestellt sind, häufig einen Röntgenopaker und den Initiator Dibenzoylperoxid auf. Vor der Zementapplikation wird die Pulverkomponente mit der flüssigen Monomerkomponente vermischt. Beim Vermischen entsteht durch Quellung der Polymere der Pulverkomponente im Methylmethacrylat ein plastisch verformbarer Teig, der eigentliche Knochenzement. Dabei reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt. Der erstarrte Polymethylmethacrylat-Knochenzement ist mechanisch stabil und kann Gelenkendoprothesen dauerhaft im Knochengewebe der Patienten befestigen.

Die Komponenten von Polymethylmethacrylat-Knochenzementen (PMMA-Knochenzementen) können in geeigneten Mischbechern mit Hilfe von Spateln vermischt werden. Nachteilig ist an dieser Vorgehensweise, dass Lufteinschlüsse im gebildeten Zementteig vorhanden sein können, die später eine Destabilisierung des ausgehärteten Knochenzements verursachen können. Aus diesem Grund wird das Vermischen von Knochenzementpulver mit der Monomerflüssigkeit in Vakuummischsystemen bevorzugt, weil durch Mischen im Vakuum Lufteinschlüsse aus dem Zementteig weitgehend entfernt werden und damit eine optimale Zementqualität erreicht wird. Im Vakuum gemischte Knochenzemente haben eine deutlich verringerte Porosität und zeigen daher verbesserte mechanische Eigenschaften. Es wurden eine Vielzahl von Vakuum-Zementiersystemen offengelegt, von denen exemplarisch folgende genannt sind: US 6 033 105 A, US 5 624 184 A, US 4 671 263 A, US 4 973 168 A, US 5 100 241 A, WO 99/67015 A1, EP 1 020 167 B1, US 5 586 821 A, EP 1 016 452 A2, DE 36 40 279 C2, WO 94/26403 A1, EP 1 005 901 A2, US 5 344 232 A.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche Full-Prepacked-Mischsysteme wurden in den Patenten EP 0 692 229 B1, DE 10 2009 031 178 B3, EP 0 875 456 B3, US 6 709 149 B1 und EP 1 140 234 B1 sowie der US 5 588 745 A vorgeschlagen.

Bei der Verwendung von Vakuum-Zementiersystemen ist es zur Applikation notwendig, den Zementteig durch Bewegung eines Kolbens aus den Kartuschen auszupressen. Dazu wurden manuelle Austragsvorrichtungen entwickelt.

In der EP 0 326 551 A1 wurde ein interessantes Hebelsystem für manuell angetriebene Vorrichtungen beschrieben. Die Idee dieses Hebelsystems basiert darauf, ein Hebelparallelogramm zu nutzen, um im Gegensatz zu einem einfachen Kipphebel die Kraft der stärksten Finger der menschlichen Hand, dem Zeigefinger und dem Mittelfinger, optimal zu nutzen.

Die einfachsten Austragsvorrichtungen basieren auf Klemmstangen mit einem darauf angeordneten kippbaren Metallplättchen, das durch eine asymmetrisch angreifende Feder verkantet und dadurch die Klemmstange einklemmt. Das kippbare Metallplättchen wird durch einen Hebel bei manueller Betätigung in Vortriebsrichtung gepresst, wobei das klemmende Metallblättchen die Klemmstange mitnimmt. Danach schiebt eine Feder das Metallplättchen zurück in seine Ausgangslage. Dieser Prozess wird wiederholt bis die Klemmstange den Förderkolben des Vakuum-Zementiersystems so weit in Richtung Kartuschenkopf gepresst hat, bis die gewünschte Knochenzementmenge aus der Kartusche gepresst ist.

Nachteilig an diesen Vorrichtungen ist, dass bei der Klemmung immer eine Rückwärtsbewegung durch Rutschen des Metallplättchens auf der Klemmstange möglich ist. Durch ein Zurückrutschen des Metallplättchens benötigt der Anwender wesentlich mehr Handbewegungen zum Auspressen des Knochenzementteigs, als eigentlich notwendig wären.

Eine Weiterentwicklung stellen Austragsvorrichtungen dar, die Zahnstangen anstelle einfacher Klemmstangen verwenden. Exemplarisch dafür ist die Austragsvorrichtung gemäß Figur 28 der Patentanmeldung US 2013 090 661 A1. Vorteilhaft ist bei solchen Vorrichtungen, dass eine Rückwärtsbewegung praktisch ausgeschlossen ist. Nachteilig ist jedoch die komplexe, aufwendige Mechanik, die eine Verwendung dieser Vorrichtung zum nur einmaligen Gebrauch hinsichtlich der relativ hohen Herstellungskosten in Frage stellt.

Die Aufgabe der Erfindung besteht darin, die Nachteile des Stands der Technik zu überwinden. Insbesondere soll eine einfach zu fertigende Austragsvorrichtung bereitgestellt werden, mit der ein Polymethylmethacrylat-Knochenzementteig aus Kartuschen von Vakuumzementiersystemen manuell ausgepresst werden kann. Die manuell angetriebene Vorrichtung soll nur zum einmaligen Gebrauch geeignet und bestimmt sein. Die Austragsvorrichtung soll weitgehend aus kostengünstigen Kunststoffteilen, die durch Kunststoffspritzgießen hergestellt werden können, und wenigen Metallelementen bestehen. Bei der manuellen Betätigung der Austragsvorrichtung soll eine Rückwärtsbewegung des Förderkolbens der Kartusche während des Auspressvorgangs infolge von elastischen Rückstellkräften der Kunststoffkartusche und des Zementteigs so weit wie möglich vermieden werden. Die zu entwickelnde Austragsvorrichtung soll nur ein einmaliges Austragen von Polymethylmethacrylat-Knochenzementteig ermöglichen. Eine Wiederverwendung und dadurch notwendige Resterilisation der Austragsvorrichtung soll konstruktiv ausgeschlossen sein.

Die Aufgaben der Erfindung werden gelöst durch eine Austragsvorrichtung für Zementkartuschen für Polymethylmethacrylat-Knochenzemente aufweisend mindestens einen manuell zu betätigenden Kipphebel, einen Adapter für eine Zementkartusche und eine in Richtung des Adapters vortreibbare Klemmstange, wobei an der Klemmstange anliegend zumindest ein Vortriebskörper angeordnet ist, der in axialer Richtung bezüglich der Achse der Klemmstange verschiebbar ist, wobei der zumindest eine an der Klemmstange anliegende Vortriebskörper mit der Klemmstange zumindest eine Kavität bildet, wobei die Kavität eine in der Vortriebsrichtung der Klemmstange geneigte Fläche oder Kante aufweist, so dass sich die Kavität im Bereich dieser Fläche oder Kante in Richtung des Adapters weitet, wobei mindestens ein kugel- oder walzenförmiger Klemmkörper in der Kavität des zumindest einen Vortriebskörpers angeordnet ist, wobei der mindestens eine Klemmkörper des zumindest einen Vortriebskörpers an mindestens einem Punkt punktuell oder entlang einer Linie die Klemmstange berührt, wobei ein elastisches Element den mindestens einen Klemmkörper des zumindest einen Vortriebskörpers aus Richtung des Adapters an die geneigte Fläche oder Kante der Kavität des zumindest einen Vortriebskörpers drückt, wobei der Kipphebel drehbar gegen die Klemmstange gelagert ist, so dass bei Betätigung des Kipphebels ein Ende des Kipphebels auf den zumindest einen axial verschiebbaren Vortriebskörper in Vortriebsrichtung der Klemmstange drückt, wobei ein Federelement entgegengesetzt zur Vortriebsrichtung der Klemmstange auf den zumindest einen Vortriebskörper drückt, zumindest ein Sicherungselement an der Klemmstange anliegt, gegen das der Vortriebskörper beweglich gelagert ist, wobei das zumindest eine an der Klemmstange anliegende Sicherungselement mit der Klemmstange zumindest eine Kavität bildet, wobei die Kavität eine zur Vortriebsrichtung der Klemmstange geneigte Fläche oder Kante aufweist, so dass sich die Kavität im Bereich dieser Fläche oder Kante in Richtung des Adapters weitet, wobei mindestens ein kugel- oder walzenförmiger Klemmkörper in der Kavität des zumindest einen Sicherungselements angeordnet ist, wobei der mindestens eine Klemmkörper des zumindest einen Sicherungselements an mindestens einem Punkt punktuell oder entlang einer Linie die Klemmstange berührt, wobei ein elastisches Element den mindestens einen Klemmkörper des zumindest einen Sicherungselements aus Richtung des Adapters an die geneigte Fläche oder Kante der Kavität des zumindest einen Sicherungselements drückt.

Bevorzugt ist der an der Klemmstange anliegende, zumindest eine Vortriebskörper in axialer Richtung bezüglich der Achse der Klemmstange gemeinsam mit der Klemmstange verschiebbar oder ausschließlich gemeinsam mit der Klemmstange verschiebbar.

Unter einem drehbar gelagerten Kipphebel wird vorliegend ein Kipphebel verstanden, der um einige Grad gegen die Klemmstange zu drehen ist. Bevorzugt ist der Kipphebel um 20° bis 70° gegen die Klemmstange zu drehen.

Unter einem walzenförmigen Klemmkörper wird vorliegend nicht nur eine Walze mit zylindrischer Geometrie verstanden. Die Walzenform kann die Geometrie einer Vielzahl verschiedener Rotationskörper aufweisen, die zum Abrollen auf der Klemmstange geeignet sind, was wiederum von der Form der Klemmstange abhängt. Eine derartige Walze kann an einem Punkt (beispielsweise Kugel als Klemmkörper auf einer zylindrischen Klemmstange) oder an mehreren Punkten (beispielsweise Walze in Form einer Sanduhr als Klemmkörper auf einer zylindrischen Klemmstange) oder entlang einer Linie (beispielsweise zylindrische Walze als Klemmkörper auf einer 4-Kant-Klemmstange) an der Klemmstange anliegen und über zumindest einem Punkt oder über die Linie auf der Klemmstange abrollen.

Anstatt einer in Richtung des Adapters vortreibbaren Klemmstange kann theoretisch auch eine in Wirkrichtung vortreibbare Klemmstange verwendet werden, wenn die Kraft, die durch den Vortrieb der Klemmstange entsteht, über Gelenke, Zahnräder oder ähnliches umgelenkt wird, dabei die Wirkrichtung geändert wird und sich dort der Adapter zum Anschließen der Zementkartusche befindet, und sich daher die Klemmstange nicht mehr geometrisch in Richtung des Adapters vortreiben lässt. Dies ist als äquivalenter Aufbau mit gleichem Wirkprinzip zu verstehen.

Mit der vorliegenden Erfindung wird auch vorgeschlagen, dass die Austragsvorrichtung ein Gehäuse mit einem Handgriff aufweist, wobei vorzugsweise das zumindest eine Sicherungselement fest mit dem Gehäuse verbunden ist und der zumindest eine Vortriebskörper beweglich gegen das Gehäuse gelagert ist. Hierdurch wird die Position des zumindest einen Sicherungselements mit dem Gehäuse festgelegt, so dass es keiner separaten Lagerung des Sicherungselements bedarf, was den Aufbau der Austragsvorrichtung vereinfacht.

Es kann auch vorgesehen sein, dass der zumindest eine Vortriebskörper die Klemmstange zumindest bereichsweise umschließt, bevorzugt zu mindestens 75% umschließt, besonders bevorzugt vollständig umschließt.

Dadurch kann die Kraft besonders einfach allseitig auf die Klemmstange wirken. Zudem wird so effektiv verhindert, dass die Austragsvorrichtung demontiert werden kann, so dass dieser Aufbau bevorzugt wird.

Dabei kann vorgesehen sein, dass der zumindest eine Vortriebskörper eine Durchführung aufweist, die bevorzugt als Röhre ausgebildet ist.

Hierdurch kann ein Verkippen und Verkanten des Vortriebskörpers auf der Klemmstange vermieden werden.

Alternativ kann vorgesehen sein, dass mehrere axial verschiebbare Vortriebskörper aus unterschiedlichen Richtungen an der Klemmstange anliegen.

Der einzelne Vortriebskörper kann so einfacher aufgebaut werden. Allerdings wird dadurch das Zusammensetzen der Austragsvorrichtung erschwert und gleichzeitig eine unerwünschte Demontage der Austragsvorrichtung erleichtert.

Mit einer Weiterbildung der Erfindung wird vorgeschlagen, dass die geneigte Fläche derart geformt ist, dass der zumindest eine Vortriebskörper und das zumindest eine Sicherungselement mindestens eine im Längsschnitt in Richtung des Adapters geneigte Kavität aufweist, bevorzugt eine keilförmige Kavität aufweist, wobei die Keilform in Richtung des Adapters geöffnet ist.

Es kann dabei auch vorgesehen sein, dass die Kavität keilförmig in Richtung der Vortriebsrichtung der Klemmstange geöffnet ist.

Bevorzugte Austragsvorrichtungen können sich auch dadurch auszeichnen, dass der zumindest eine kugel- oder walzenförmige Klemmkörper um zumindest eine Achse drehbar in der Kavität des zumindest einen Vortriebskörpers und/oder des zumindest einen Sicherungselements angeordnet oder gelagert ist.

Hierdurch kann eine sehr effektive und stabile Blockade der Klemmstange erreicht werden, da sich die abrollenden Klemmkörper je nach Abrollrichtung zwischen der geneigten Fläche oder Kante der Kavität und der Klemmstange festziehen können und bei umgekehrter Drehung ein Abrollen auf der Klemmstange erlauben, so dass der zumindest eine Vortriebskörper beziehungsweise das zumindest eine Sicherungselement über die Klemmstange rutschen kann. Dabei reichen bereits sehr kleine Winkel für die Drehung des kugel- oder walzenförmigen Klemmkörpers aus, um die Funktion des Klemmkörpers zu gewährleisten. Bevorzugt kann der zumindest eine kugel- oder walzenförmige Klemmkörper auf der Klemmstange um einen Winkel von zumindest 1° abrollen.

Gemäß einer Weiterbildung der erfindungsgemäßen Austragsvorrichtung kann vorgesehen sein, dass die Kavität, die durch den zumindest einen Vortriebskörper und die Klemmstange gebildet ist, und/oder die Kavität, die durch das zumindest eine Sicherungselement und die Klemmstange gebildet ist, als Trichter ausgebildet ist oder sind, wobei der Trichter die Klemmstange umschließt.

Hierdurch können die Klemmkörper allseitig in dem Trichter und mit der Klemmstange verkeilen beziehungsweise verklemmen und dadurch mit der Klemmstange blockieren beziehungsweise an die Klemmstange anklemmen.

Bevorzugte Ausführungsformen können vorsehen, dass die Klemmkörper aus Keramik oder Metall gebildet sind und besonders bevorzugt aus Stahl und/oder Wolfram.

Besonders bevorzugt bestehen die Klemmkörper aus einem Material, das härter ist als das Material des zumindest einen Vortriebskörpers, des zumindest einen Sicherungselements und der Klemmstange.

Hierdurch wird erreicht, dass sich die Klemmkörper in die Klemmstange und den zumindest einen Vortriebskörper und das zumindest eine Sicherungselement eindrücken können, nachfolgend aber immer noch auf der Klemmstange abrollen können.

Des Weiteren kann vorgesehen sein, dass die geneigte Fläche oder Kante der Kavität des zumindest einen Vortriebskörpers und/oder des zumindest einen Sicherungselements aus Metall oder aus einem Metall-Kunststoffverbund besteht.

Somit kann sichergestellt werden, dass sich der festziehende Klemmkörper wieder von der geneigten Fläche oder Kante des zumindest einen Vortriebskörpers und/oder des zumindest einen Sicherungselements wieder lösen kann.

Ferner kann vorgesehen sein, dass das zumindest eine Sicherungselement auf der Klemmstange vor oder auch hinter dem zumindest einen Vortriebskörper in Bezug zur Vortriebsrichtung der Klemmstange angeordnet ist. Bevorzugt ist das zumindest eine Sicherungselement auf der Klemmstange zwischen dem zumindest einen Vortriebskörper und dem Adapter angeordnet.

Bevorzugte Austragsvorrichtungen können vorsehen, dass die Klemmkörper und die Klemmstange eine Härte von mindestens 50 HRC haben.

Hierdurch kann ein mehrfaches Festziehen und wieder lösen der Klemmkörper von der Klemmstange sichergestellt werden.

Mit einer Weiterbildung der vorliegenden Erfindung wird vorgeschlagen, dass die elastischen Elemente, die den zumindest einen Klemmkörper des zumindest einen Vortriebskörpers und den zumindest einen Klemmkörper des zumindest einen Sicherungselements an die geneigten Flächen und/oder Kanten der Kavitäten des zumindest einen Vortriebskörpers und des zumindest einen Sicherungselements drücken, elastisch Körper sind, die vorzugsweise die Kavitäten in Richtung des Adapters verschließen.

Hierdurch wird sichergestellt, dass die Klemmkörper die zumindest eine Vortriebsrichtung und das zumindest eine Sicherungselement schnell gegen die Klemmstange blockieren können, indem sie durch die elastischen Körper geeignet positioniert werden und in dieser Position auch gegen möglicherweise wirkende Kräfte gehalten werden.

Mit einer bevorzugten Weiterbildung der vorliegenden Austragsvorrichtung wird vorgeschlagen, dass der Kraftschluss des zumindest einen Klemmkörpers des zumindest einen Vortriebskörpers mit der Klemmstange und der Kraftschluss des zumindest einen Klemmkörpers des zumindest einen Sicherungselements mit der Klemmstange nicht gleichzeitig lösbar sind, so dass die Klemmstange mit der Austragsvorrichtung nur unidirektional vorzutreiben ist.

Dadurch wird durch eine einfache konstruktive Maßnahme erreicht, dass die Klemmstange nicht wieder zurück geschoben werden kann, um die Austragsvorrichtung erneut zu verwenden. Hiermit wird erreicht, dass nur sterile Austragsvorrichtungen eingesetzt werden können und es nicht zu einer versehentlichen Verunreinigung des OP-Bereichs oder zu einer Infektion am Patienten kommt.

Die der Erfindung zugrundeliegenden Aufgaben werden auch gelöst durch ein Verfahren zum Vortreiben einer Klemmstange mit einem Kipphebel bei dem durch manuelle Krafteinwirkung auf den Kipphebel zumindest ein an der Klemmstange anliegender Vortriebskörper in einer ersten axialen Richtung der Klemmstange bewegt wird,
wobei zumindest ein Klemmkörper durch die Kraft der Bewegung gegen eine geneigte Fläche oder Kante der Kavität an dem zumindest einen Vortriebskörper und die Klemmstange gedrückt wird, wobei der Klemmkörper in einer Kavität des zumindest einen Vortriebskörpers angeordnet ist, wobei die Kavität durch den zumindest einen Vortriebskörper und die Klemmstange gebildet wird, und wobei der Klemmkörper die Klemmstange an wenigstens einem Punkt oder entlang einer Linie berührt, und wobei der zumindest eine Klemmkörper und der zumindest eine Vortriebskörper durch den Druck derart kraftschlüssig mit der Klemmstange verbunden sind, dass die Klemmstange mit dem Vortriebskörper bewegt wird,
wobei sobald die manuelle Krafteinwirkung auf den Kipphebel reduziert wird oder beendet wird der zumindest Vortriebskörper durch ein Federelement in die entgegengesetzte axiale zweite axiale Richtung gedrückt wird, wobei der Kipphebel wieder in die Ausgangsstellung gekippt wird und wobei sich durch die umgekehrte Krafteinwirkung der zumindest eine Klemmkörper von der geneigten Fläche oder Kante des zumindest einen Vortriebskörpers in der Kavität löst und dadurch über die Klemmstange gleitet und/oder abrollt,
wobei eine umgekehrte Bewegung der Klemmstange in die zweite axiale Richtung durch zumindest ein an der Klemmstange anliegendes Sicherungselement blockiert wird, wobei zumindest ein Klemmkörper des Sicherungselements durch die umgekehrte Bewegung der Klemmstange gegen eine geneigte Fläche oder Kante der Kavität an dem zumindest einen Sicherungselement und die Klemmstange gedrückt wird, wobei der Klemmkörper in einer Kavität des zumindest einen Sicherungselements angeordnet ist, die durch das zumindest eine Sicherungselement und die Klemmstange gebildet wird, und wobei der Klemmkörper die Klemmstange an wenigstens einem Punkt oder entlang einer Linie berührt, und der zumindest eine Klemmkörper und das zumindest eine Sicherungselement durch den Druck auf den Klemmkörper derart kraftschlüssig mit der Klemmstange verbunden sind, dass die Klemmstange sich nicht gegen das zumindest eine Sicherungselement bewegt.

Dabei kann vorgesehen sein, dass sich bei einer Bewegung der Klemmstange in die erste axiale Richtung der zumindest eine Klemmkörper des zumindest einen Sicherungselements von der geneigten Fläche oder Kante der Kavität des zumindest einen Sicherungselements löst und die Klemmstange dadurch gegen das zumindest eine Sicherungselement bewegt wird.

Hierdurch wird sichergestellt, dass die Klemmstange problemlos vorgetrieben werden kann.

Des Weiteren kann vorgesehen sein, dass der zumindest eine Klemmkörper des zumindest einen Vortriebskörpers und der zumindest eine Klemmkörper des zumindest einen Sicherungselements durch Kugeln und/oder Walzen gebildet werden, die auf der Klemmstange zumindest geringfügig abrollen.

Bevorzugt rollen die Kugeln und/oder Walzen um wenigstens 1° auf der Klemmstange ab.

Ferner kann vorgesehen sein, dass die geneigte Fläche oder Kante der Kavität des zumindest einen Vortriebskörpers und/oder des zumindest einen Sicherungselements im Querschnitt keilförmig ist.

Hierdurch kann ein besonders einfacher Aufbau des zumindest einen Vortriebskörpers und/oder des zumindest einen Sicherungselements erreicht werden. Die Neigung der Fläche oder Kante beträgt bevorzugt zwischen 10° und 30°, besonders bevorzugt zwischen 15° und 25° bezüglich der Klemmstange.

Auch kann vorgesehen sein, dass beim Kippen des Kipphebels durch manuelle Krafteinwirkung das Federelement komprimiert wird.

Hierdurch wird das Federelement für die anschließende Rückstellung des zumindest einen Vortriebskörpers gespannt.

Erfindungsgemäß kann ferner vorgesehen sein, dass ein elastisches Element den mindestens einen Klemmkörper des zumindest einen Sicherungselements aus Richtung des Adapters an die geneigte Fläche oder Kante der Kavität des zumindest einen Sicherungselements drückt. Das Gleiche gilt auch für den Vortriebskörper.

Hierdurch wird eine geeignete Positionierung des Klemmkörpers erreicht.

Erfindungsgemäße Verfahren können sich auch dadurch auszeichnen, dass die Bewegung mehrfach wiederholt wird und dabei die Klemmstange schubweise vorgetrieben wird und dabei schubweise ein Knochenzement aus einer Kartusche ausgetrieben wird, die zuvor über einen Adapter mit einer Austragsvorrichtung verbunden wurde, wobei die Austragsvorrichtung den Adapter, den Kipphebel, die Klemmstange, den zumindest einen Vortriebskörper und das zumindest eine Sicherungselement umfasst.

Schließlich kann vorgesehen sein, dass das Verfahren durch Verwendung oder unter Anwendung einer erfindungsgemäßen Austragsvorrichtung durchgeführt wird.

Der Erfindung liegt die überraschende Erkenntnis zugrunde, dass es durch die Verwendung eines Sicherungselements und eines manuell angetriebenen Vortriebskörpers, die jeweils einen beweglichen Klemmkörper in einer angeschrägten oder keilförmigen Kavität aufweisen und die relativ zueinander beweglich an einer Klemmstange angeordnet sind, gelingt, einen einfachen aber effektiven unidirektionalen Vortrieb der Klemmstange zu erreichen, ohne dass die Klemmstange aufgrund elastischer Kräfte der PMMA-Zementkartusche wieder zurückgetrieben werden könnte. Zudem können die Klemmkörper bei geeignetem Aufbau sich aus den Kavitäten lösen, wenn die Klemmstange entfernt wird, was ein anschließendes erneutes Zusammensetzen zumindest erschwert oder unmöglich macht. Hierdurch wird die Wiederverwendbarkeit eingeschränkt, so dass die Verwendung nicht sterilisierter Austragsvorrichtungen unterbunden werden kann. Gleichzeitig ist der gesamte Aufbau der Austragsvorrichtung kostengünstig zu realisieren, so dass das Wegwerfprodukt nicht zu teuer in der Herstellung ist. Zudem ist es aufgrund der nur unidirektional vorzutreibenden Klemmstange nicht möglich, die Austragsvorrichtung wiederzuverwenden.

Es wurde also überraschend gefunden, dass mit der erfindungsgemäßen Austragsvorrichtung trotz der Verwendung einfacher, kostengünstiger Klemmstangen und von kostengünstigen Kunststoffteilen ein Austrag von Polymethylmethacrylat-Knochenzementteig möglich ist, ohne dass unerwünschte Rückwärtsbewegungen der Austragskolben während des Austragens von Polymethylmethacrylat-Knochenzementteig aus Vakuum-Zementiersystemen auftreten. Weiterhin wurde gefunden, dass es ohne Zerstörung der Austragsvorrichtung nicht möglich ist, diese in den Ausgangszustand zu versetzen, so dass eine Wiederverwendung der Vorrichtung nach erfolgtem Austrag ausgeschlossen ist.

Eine erfindungsgemäße Austragsvorrichtung kann beispielsweise zusammengesetzt sein aus mindestens einem manuell zu betätigenden Kipphebel, einer Klemmstange, einem Adapter für die Zementkartusche und einem Gehäuse mit Handgriff. Die Austragsvorrichtung kann beispielsweise dadurch charakterisiert sein, dass
a) auf der Klemmstange ein axial verschiebbarer Vortriebskörper angeordnet ist, der die Klemmstange zumindest bereichsweise umschließt,
b) der Vortriebskörper mindestens eine im Längsschnitt keilförmige oder geneigte Kavität besitzt, wobei die Keilform oder Neigung in Richtung der Bewegungsrichtung der Klemmstange geöffnet ist,
c) mindestens ein kugel- oder walzenförmiger Klemmkörper in der im Längsschnitt keilförmigen oder geneigten Kavität angeordnet ist, wobei der Klemmkörper mindestens punktuell oder entlang einer Linie die Klemmstange berührt,
d) ein elastisches Element den mindestens einen Klemmkörper in die im Längsschnitt keilförmige oder geneigte Kavität des Vortriebskörpers drückt,
e) der Kipphebel drehbar so im Gehäuse angeordnet ist, dass bei Betätigung des Kipphebels ein Ende des Kipphebels auf den axial verschiebbaren Vortriebskörper in Vortriebsrichtung der Klemmstange gedrückt wird,
f) ein Federelement entgegengesetzt zur Vortriebsrichtung der Klemmstange auf den Vortriebskörper drückt,
g) mit dem Gehäuse ein Sicherungselement verbunden ist, dass die Klemmstange zumindest bereichsweise umschließt, wobei das Sicherungselement mindestens eine keilförmige oder geneigte Kavität besitzt, die in Richtung der Vortriebsrichtung der Klemmstange geöffnet ist,
h) mindestens einem kugel- oder walzenförmigen Klemmkörper der in der im Längsschnitt keilförmigen oder geneigten Kavität angeordnet ist, wobei der kugel- oder walzenförmige Klemmkörper mindestens punktuell oder entlang einer Linie die Klemmstange berührt und dass
i) mindestens ein elastisches Element den Klemmkörper in die keilförmige oder geneigte Kavität des Sicherungselements drückt.

Das Gehäuse und der Adapter für die Zementkartusche bestehen vorzugsweise aus Kunststoff. Die Klemmstange ist aus Metall, vorzugsweise aus Stahl gefertigt. Der Kipphebel besteht entweder aus Kunststoff, Aluminiumlegierungen oder Stahl.

Der Kipphebel ist bevorzugt als Hebelsystem gemäß der EP 0 326 551 A1 ausgebildet. Bei diesem Hebelsystem wird ein manuell zu betätigendes Hebelparallelogramm verwendet, das eine kraftvolle Betätigung des Hebels durch den Zeigefinger und Mittelfinger ermöglicht.

Die Durchführung des Vortriebskörpers ist bevorzugt als Röhre ausgebildet. Dadurch ist ein Verkippen oder Verkanten des Vortriebskörpers auf der Klemmstange ausgeschlossen.

Die im Längsschnitt keilförmige oder geneigte Kavität des Vortriebskörpers und auch des Sicherungselements ist bevorzugt als Trichter ausgebildet. Daneben ist es auch möglich, die Kavität als Ausschnitte eines Trichters auszubilden. Ebenfalls ist es möglich, die Kavität als keilförmiges Prisma zu gestalten.

Die Klemmkörper sind aus Metall und/oder Keramik gebildet. Bevorzugt bestehen die Klemmkörper aus Stahl oder aus Wolfram. Daneben ist es auch möglich, dass die Klemmkörper aus harten keramischen Werkstoffen, wie Siliziumcarbid, Wolframkarbid oder Borcarbid gefertigt sind.

Die im Längsschnitt keilförmige oder geneigte Kavität besteht aus Metall oder aus einem Metall-Kunststoffverbund. Wichtig ist dabei, dass die Klemmkörper in einem harten Widerlager gelagert sind, damit bei der Klemmung des Vortriebskörpers und auch des Sicherungselements die Klemmkörper nicht den Vortriebskörper und auch nicht das Sicherungselement deformieren können. Eine Deformation des Vortriebskörpers und auch des Sicherungselements könnte zu einer Blockierung der Bewegung der Klemmstange führen. Kleinere oder geringere plastische Deformationen stören hingegen nicht, sofern dadurch nicht die Funktion der Austragsvorrichtung beeinträchtigt wird.

Das Sicherungselement kann auf der Klemmstange vor oder auch nach dem Vortriebskörper in Bezug zur Vortriebsrichtung angeordnet sein.

Die Klemmkörper und die Klemmstange haben eine Härte von mindestens 50 HRC. Bevorzugt beträgt die Härte mindestens 56 HRC. Unter dem Begriff "HRC" wird die Rockwell-Härte nach der Skala C gemäß der DIN EN ISO 6508-1 verstanden.

Ein erfindungsgemäßes Verfahren zum Austragen von Polymethylmethacrylat-Knochenzement mit einer erfindungsgemäßen Austragsvorrichtung kann beispielsweise dadurch charakterisiert werden, dass in einem Schritt a) manuell der Kipphebel gegen den in einer Ausgangsstellung befindlichen Vortriebskörper bewegt wird, wobei der Vortriebskörper durch Klemmung mit dem mindestens einen Klemmkörper gegen die Klemmstange und die im Längsschnitt keilförmige Kavität die Klemmstange klemmt und in Vortriebsrichtung bewegt, wobei gleichzeitig das Federelement komprimiert wird,

in einem Schritt b) nach Beendigung der Vortriebsbewegung des Kipphebels der Vortriebskörpers durch das komprimierte Federelement auf der Klemmstange zurück in die Ausgangsstellung gedrückt wird,

in einem Schritt c) das Sicherungselement durch Klemmung des mindestens einen Klemmkörpers gegen die Klemmstange und die im Längsschnitt keilförmige Kavität gegen eine Bewegung entgegengesetzt zur Vortriebsrichtung gesichert wird und dass die Schritte a bis c wiederholt werden.

Im Folgenden werden weitere Ausführungsbeispiele der Erfindung anhand von sechs schematisch dargestellten Figuren erläutert, ohne jedoch dabei die Erfindung zu beschränken. Dabei zeigt:
- Figur 1:: eine schematische Querschnittansicht einer erfindungsgemäßen Austragsvorrichtung;
- Figur 2:: eine schematische perspektivische Ansicht der Austragsvorrichtung nach Figur 1;
- Figur 3:: eine schematische perspektivische Seitenansicht der Austragsvorrichtung nach den Figuren 1 und 2;
- Figur 4:: eine schematische perspektivische Ansicht von schräg unten auf die Austragsvorrichtung nach den Figuren 1 bis 3;
- Figur 5:: eine schematische Aufsicht von oben auf die Austragsvorrichtung nach den Figuren 1 bis 4; und
- Figur 6:: eine schematische perspektivische Querschnittansicht eines Teils einer alternativen erfindungsgemäßen Austragsvorrichtung.

In den Figuren werden auch bei unterschiedlichen Ausführungsformen die gleichen Bezugszeichen verwendet.

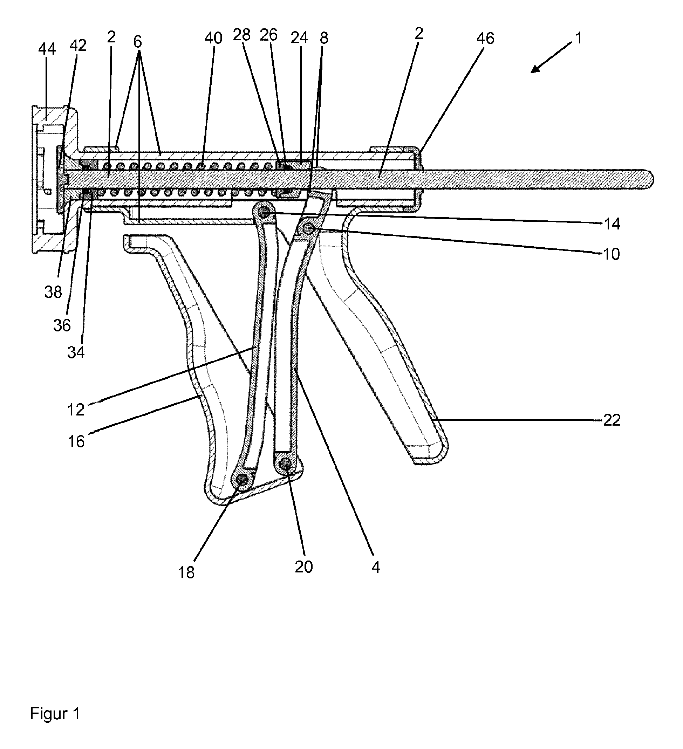

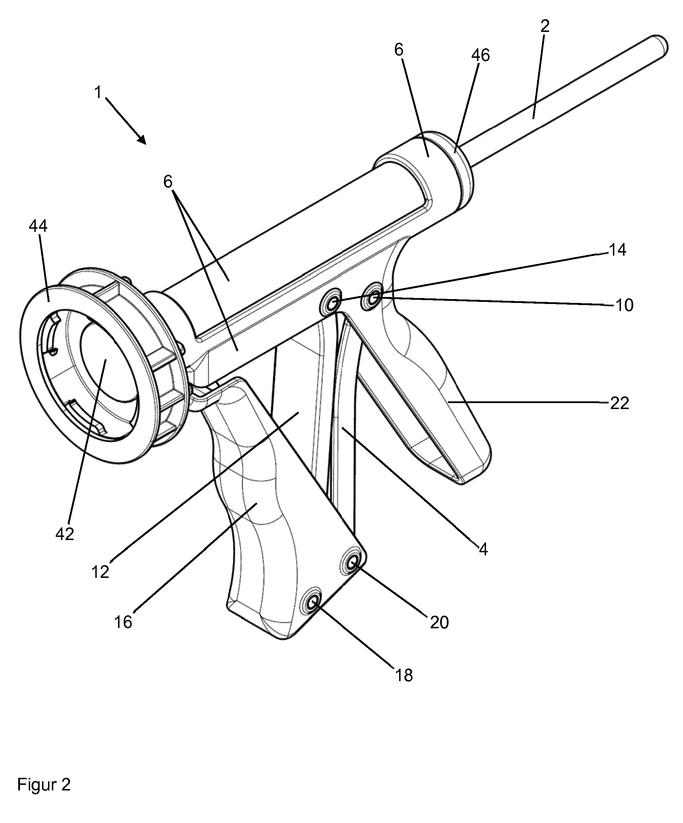

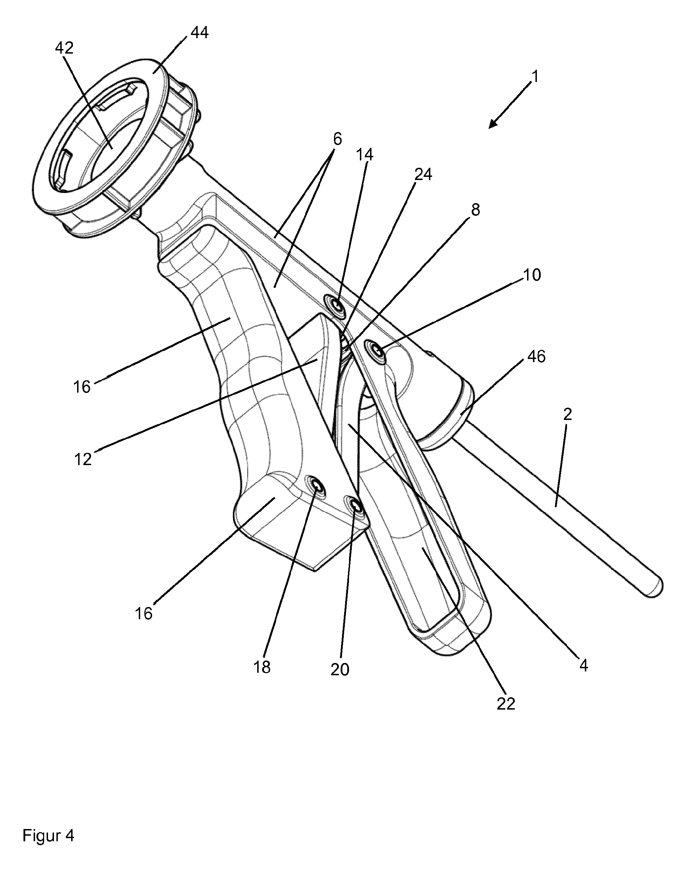

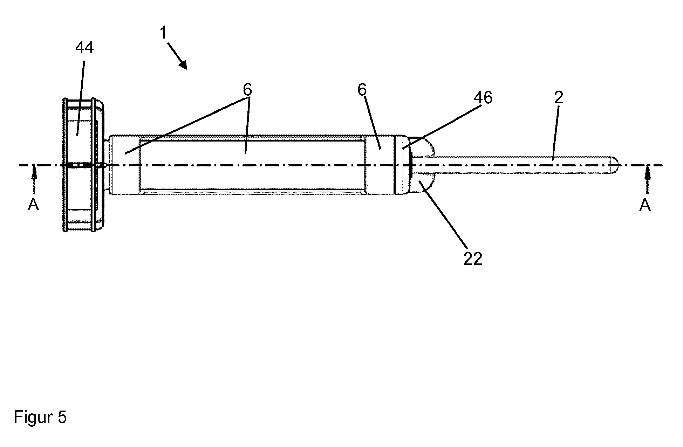

Figur 1 zeigt eine Austragsvorrichtung 1 mit einer Klemmstange 2, die nach vorne (in Figur 1 links) vortreibbar ist. Die Figuren 2 bis 4 zeigen hierzu perspektivische Ansichten. Eine Aufsicht auf die Austragsvorrichtung 1 ist in Figur 5 dargestellt. Der in Figur 1 dargestellte Querschnitt ist in Figur 5 durch eine Strich-Punkt-Linie A gekennzeichnet. Die Querschnittansicht nach Figur 1 entspricht also dem Schnitt entlang der Linie A-A in Figur 5.

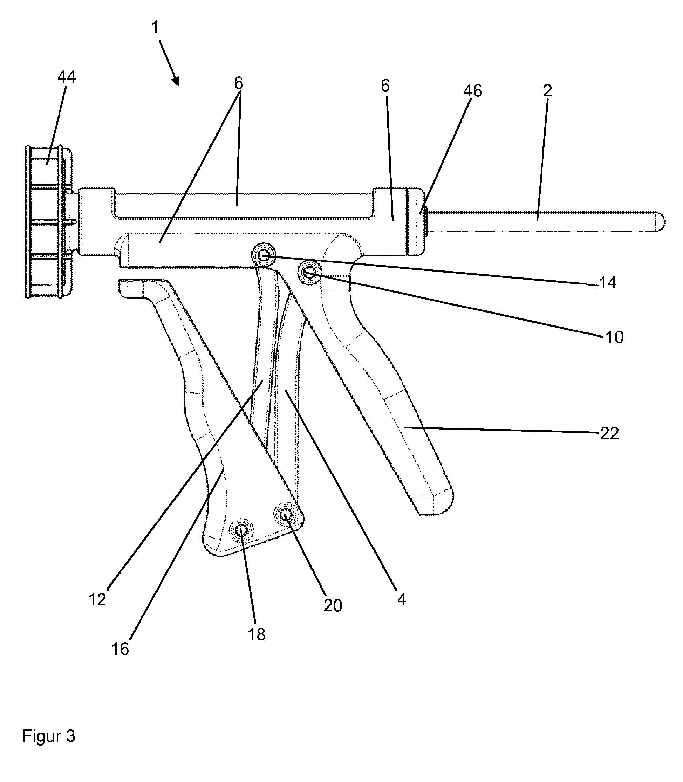

Die Klemmstange 2 kann mit Hilfe eines Kipphebels 4 aus einem stabilen Kunststoff oder alternativ aus Stahl bewegt werden. Die Austragsvorrichtung 1 wird von einem mehrteiligen Gehäuse 6 aus Kunststoff weitgehend umschlossen, damit der Innere Aufbau der Austragsvorrichtung 1 nicht offenliegt. Die Gehäuseteile 6 können als Spritzgussteile gefertigt werden. Der Kipphebel 4 endet in einem schwenkbaren Kopf 8, mit dem die Bewegung des Kipphebels 4 ins Innere des Gehäuses 6 der Austragsvorrichtung 1 übersetzt wird. Dazu ist der Kipphebel 4 über eine Achse 10 mit dem Gehäuse 6 verbunden. Bei einer Drehung des Kipphebels 4 um die Achse 10 (bei der Aufsicht auf den Querschnitt nach Figur 1 und der Seitenansicht nach Figur 3 entgegen des Uhrzeigersinns) wird der Kopf 8 des Kipphebels 4 aufgrund der Hebelwirkung mit großer Kraft nach vorne (in den Figuren 1, 3 und 5 nach links) gedrückt.

Der Kipphebel 4 wird bedient, indem eine Strebe 12 bewegt wird, die über eine Achse 14 mit dem Gehäuse 6 und mit einem Abzug 16 über eine Achse 18 verbunden ist. Der Kipphebel 4 ist ebenfalls über eine Achse 20 mit dem Abzug 16 verbunden. Der Abzug 16 lässt sich aufgrund dieses Aufbaus parallel zu einem Griff 22 bewegen. Der Griff 22 ist als Teil des Gehäuses 6 aus Kunststoff gefertigt. Durch den Aufbau mit dem Kipphebel 4 und der Strebe 12 sowie deren Verbindungen / Achsen 10, 14, 18, 20 zum Gehäuse 6 und dem Abzug 16 kann die volle Höhe des Abzugs 16 verwendet werden, um einen Druck auf den Kipphebel 4 auszuüben. Dadurch lässt sich der Kipphebel 4 mit der Kraft der ganzen Hand und insbesondere auch des Zeigefingers und des Mittelfingers bedienen, wobei der Griff 22 mit der gleichen Hand gehalten wird. Die gesamte Austragsvorrichtung 1 kann also leicht mit einer Hand gehalten und bedient werden. Ein solcher Aufbau ist im Detail auch in der EP 0 326 551 A1 beschrieben.

Mit dem Kopf 8 des Kipphebels 4 wird ein Vortriebskörper 24 vorangetrieben (in den Figuren 1, 3 und 5 nach links verschoben). Der Vortriebskörper 24 umschließt die Klemmstange 2 und weist an der Innenseite eine Ausnehmung auf, die mit der Klemmstange 2 eine Kavität bildet. In der Kavität sind mehrere Kugeln 26 aus Stahl oder Keramik als Klemmkörper 26 angeordnet, die an der Klemmstange 2 anliegen.

Die Kavität beziehungsweise die Ausnehmung des Vortriebskörpers 24 hat eine nach vorne (in den Figuren 1, 3 und 5 nach links) geneigte Grenzfläche, die nach Art eines Trichters ebenfalls umlaufend um die Klemmstange 2 ausgebildet ist. In der Kavität, die durch den Trichter und die Klammstange 2 sowie einen Verschluss 28 auf der Vorderseite (in den Figuren 1, 3 und 5 links) begrenzt ist, sind eine Vielzahl von Kugeln 26 als Klemmkörper 26 nach Art eines Kugellagers angeordnet. Der Verschluss 28 ist elastisch und drückt die Kugeln 26 leicht gegen die geneigte Wandung der Kavität. Die Neigung der Trichterwand beträgt etwa 20° gegenüber der Achse der Klemmstange 2.

Wenn der Kopf 8 des Kipphebels 4 aufgrund einer Drehung um die Achse 10 auf den Vortriebskörper 24 drückt, dann werden die Kugeln 26 gegen die geneigte Wand der Kavität und die Klemmstange 2 gepresst. Dadurch blockieren die Kugeln 26 beziehungsweise die Klemmkörper 26 und der Vortriebskörper 24 verklemmt sich beziehungsweise verkeilt sich mit der Klemmstange 2. Durch die geringe Auflagefläche (Auflagepunkte) der Kugeln 26 auf der Klemmstange 2 beziehungsweise auf der Wandung der Kavität. entsteht ein sehr großer Druck an den Auflageflächen, der ausreicht, um ein Vorschieben des Vortriebskörpers 24 auf der Klemmstange 2 zu verhindern.

An der Vorderseite der Austragsvorrichtung 1 (in den Figuren 1, 3 und 5 links) ist ein Sicherungselement 34 vorgesehen, das in seinem Aufbau dem Vortriebskörper 24 gleicht, wobei das Sicherungselement 34 fest mit dem Gehäuse 6 verbunden ist, während der Vortriebskörper 24 im Gehäuse 6 beweglich gelagert ist.

Das Sicherungselement 34 umschließt die Klemmstange 2 und weist an der Innenseite eine Ausnehmung auf, die mit der Klemmstange 2 eine Kavität bildet. In der Kavität sind mehrere Kugeln 36 aus Stahl oder Keramik als Klemmkörper 36 angeordnet, die an der Klemmstange 2 anliegen. Die Kavität beziehungsweise die Ausnehmung des Sicherungselements 34 hat eine nach vorne (in den Figuren 1, 3 und 5 nach links) geneigte Grenzfläche, die nach Art eines Trichters ebenfalls umlaufend um die Klemmstange 2 ausgebildet ist. In der Kavität, die durch den Trichter und die Klammstange 2 sowie einen Verschluss 38 auf der Vorderseite (in den Figuren 1, 3 und 5 links) begrenzt ist, sind eine Vielzahl von Kugeln 36 als Klemmkörper 36 nach Art eines Kugellagers angeordnet. Der Verschluss 38 ist elastisch und drückt die Kugeln 36 leicht gegen die geneigte Wandung der Kavität. Die Neigung der Trichterwand beträgt etwa 20° gegenüber der Achse der Klemmstange 2.

Wenn die Klemmstange 2 mit dem Kipphebel 4 und dem Vortriebskörper 24 nach vorne getrieben wird, schiebt sich die Klemmstange 2 durch das Sicherungselement 34, da die Kugeln 36 bei dieser Bewegung auf der Klemmstange 2 aufgrund der Neigung der Trichterwand abrollen können. Gleichzeitig wird eine elastische Feder 40 gespannt beziehungsweise elastisch komprimiert, die zwischen dem Vortriebskörper 24 und dem Sicherungselement 34 in dem Gehäuse 6 angeordnet ist.

Wenn die Kraft auf den Kipphebel 4 nachlässt oder wegfällt, wird der Vortriebskörper 24 durch die gespannte Feder 40 in die entgegengesetzte Richtung nach hinten (in den Figuren 1, 3 und 5 nach rechts) gedrückt. Bei dieser Bewegung können die Kugeln 26 des Vortriebskörpers 24 aufgrund der Neigung der Trichterwand der Kavität auf der Stange 2 abrollen. Dadurch kann der Vortriebskörper 24 auf der Klemmstange 2 verschoben werden.

Gleichzeitig ist es nicht möglich, die Klemmstange 2 wieder nach hinten (in den Figuren 1, 3 und 5 nach rechts) ins Gehäuse 6 zu drücken, da diese Bewegung aufgrund der Neigung des Trichters des Sicherungselements 34 mit den Klemmkörpern 36 beziehungsweise den Kugeln 36 blockiert wird. Wenn also beim Auspressen von Knochenzement aus einer Kartusche (nicht gezeigt) elastische Kräfte auftreten, die einen Gegendruck auf die Klemmstange 2 ausüben, kann die Klemmstange 2 nicht wieder in das Gehäuse 6 zurück gedrückt werden.

An der Vorderseite ist an der Klemmstange 2 ein Stempel 42 befestigt, der zum Vortreiben eines Förderkolbens einer Kartusche (nicht gezeigt) vorgesehen ist. An der Vorderseite der Austragsvorrichtung 1 befindet sich ein Adapter 44 mit einem Bajonett-Verschluss zum Anschließen einer Zementkartusche. Die Zementkartusche (nicht gezeigt) kann an dem Adapter 44 befestigt werden, wobei der Boden der befestigten Kartusche den Förderkolben beinhaltet, der mit dem Stempel 42 in die Kartusche hineingedrückt werden kann. Durch das Vortreiben der Klemmstange 2 drückt der Stempel 42 den Förderkolben in die Kartusche, wobei dadurch der Inhalt der Kartusche (beispielsweise ein medizinischer PMMA-Knochenzement) aus der Kartusche durch eine dem Förderkolben gegenüberliegende Kartuschenöffnung herausgedrückt wird.

Auf der Rückseite ist die Austragsvorrichtung durch eine Kappe 46 verschlossen. Die Kappe 46 weist eine Durchführung für die Klemmstange 2 auf und kann als Teil des Gehäuses 6 betrachtet werden.

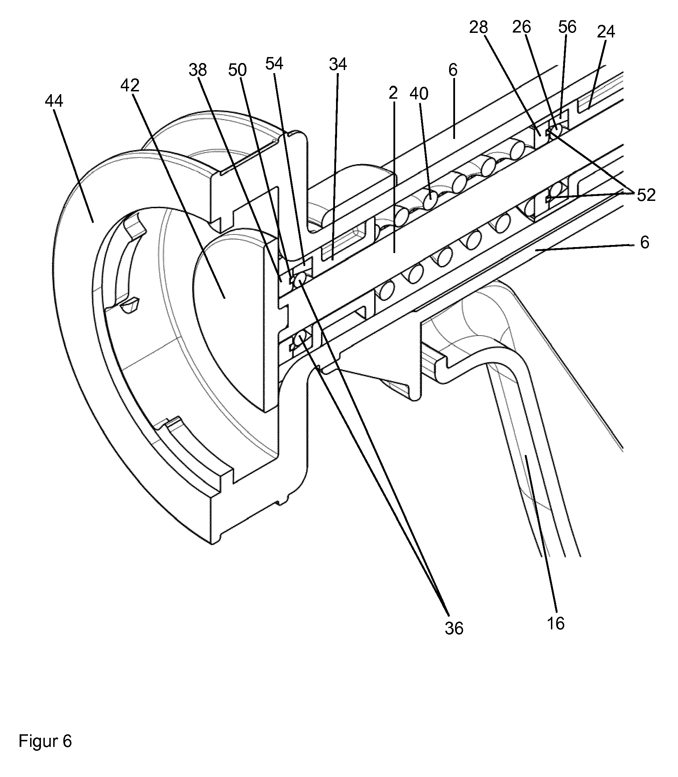

Figur 6 zeigt eine schematische perspektivische Querschnittansicht eines Teils einer alternativen erfindungsgemäßen Austragsvorrichtung. Die Austragsvorrichtung entspricht bis auf einige Details zu dem Vortriebskörper 24 und dem Sicherungselement 34 den Ausführungen nach den Figuren 1 bis 5.

In der Austragsvorrichtung ist eine Stange 2 in Längsrichtung beweglich gelagert. Die Austragsvorrichtung weist ein Gehäuse 6 aus Kunststoff auf, in dem die Stange 2 beweglich gelagert ist. Bedient wird diese Austragsvorrichtung mit einem Abzug 16, der analog zu der Ausführung nach Figur 1 bis 5 im Bereich eines Griffs (nicht gezeigt) an der Unterseite (in Figur 6 unten) der Austragsvorrichtung angeordnet ist und der analog dazu über ein Hebel-Parallelogramm bedient wird. Zum Vortrieb der Stange 2 ist ein Vortriebskörper 24 vorgesehen, der eine Kavität aufweist, in der Kugeln 26 oder Walzen 26 gelagert sind. Auf der Vorderseite (in Figur 6 links) ist die Kavität des Vortriebkörpers 24 mit einem Verschluss 28 verschlossen. Analog dazu ist auch ein Sicherungselement 34 aufgebaut, das ebenfalls eine Kavität mit der Stange 2 bildet, in dem sich Walzen 36 oder Kugeln 36 befinden. Das Sicherungselement 34 ist mit dem Gehäuse 6 fest verbunden, während der Vortriebskörper 24 beweglich im Gehäuse 6 gelagert ist. Auch die Kavität des Sicherungselements 34 ist auf der Vorderseite mit einem Verschluss 38 verschlossen.

Zwischen dem Vortriebskörper 24 und dem Sicherungselement 34 ist eine elastische Feder 40 aus Stahl angeordnet, die den Vortriebskörper 24 von dem Sicherungselement 34 weg (nach hinten - in Figur 6 rechts) drückt. Die Stange 2 endet an der Vorderseite in einem Stempel 42, der in einem Adapter 44 mit einem Bajonett-Anschluss angeordnet ist.

In dem Verschluss 38 befindet sich eine umlaufende Nut in der ein elastischer O-Ring 50 aus Gummi angeordnet ist. Analog dazu befindet sich in dem Verschluss 28 eine umlaufende Nut in der ein elastischer O-Ring 52 aus Gummi angeordnet ist. Ferner ist in der Kavität des Sicherungselements 34 ein Konus 54 als Einsatz vorgesehen und in der Kavität der Vortriebkörpers 24 ebenso ein Konus 56 angeordnet. Die Konusse 54, 56 bilden geneigte Wandungen, gegen die die Kugeln 26, 36 mit Hilfe der elastischen O-Ringe 50, 52 gedrückt werden. Durch die Neigung des Konus 56 des Vortriebskörpers 24 werden die Kugeln 26 gegen die Stange 2 gepresst, wenn der Vortriebskörper 24 in Richtung des Sicherungselements 34 vorgetrieben wird, so dass die Stange 2 mit dem Vortriebskörper 24 mit bewegt wird. Durch die Neigung des Konus 54 des Sicherungselements 34 werden die Kugeln 36 gegen die Stange 2 gepresst, wenn die Stange 2 nach hinten (in Figur 6 nach rechts) bewegt werden soll. Dadurch wird die Bewegung der Stange 2 mit dem Vortriebskörper 24 nach hinten (in Figur 6 nach rechts) verhindert.

Der Vortriebskörper 24 ist über den Abzug 16 nach vorne in Richtung des Sicherungselements 34 zu treiben. Dabei wird die Stange 2 nach vorne geschoben und die Feder 40 gespannt. Beim Loslassen des Abzugs 16 wird der Vortriebskörper 24 wieder von dem Sicherungselement 34 weg gedrückt. Eine Rückwärtsbewegung der Stange 2 wird dabei von dem Sicherungselement 34 verhindert.

Durch den Aufbau beider Ausführungsformen nach den Figuren 1 bis 6 kann die Stange 2 nur nach vorne getrieben werden, während eine Rückwärtsbewegung der Stange 2 nicht möglich ist. Dadurch, dass keine Möglichkeit gegeben wird, die Stange 2 in die Ausgangsposition zurück zu überführen, wird eine ungewollte erneute Verwendung der Austragsvorrichtung baulich ausgeschlossen.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Austragsvorrichtung
- 2: Klemmstange
- 4: Kipphebel
- 6: Gehäuse
- 8: Kopf des Kipphebels
- 10: Achse
- 12: Strebe
- 14: Achse
- 16: Abzug
- 18: Achse
- 20: Achse
- 22: Griff
- 24: Vortriebskörper
- 26: Kugel / Klemmkörper
- 28: Verschluss
- 34: Sicherungselement
- 36: Kugel / Klemmkörper
- 38: Verschluss
- 40: Feder
- 42: Stempel
- 44: Adapter / Bajonettanschluss
- 46: Kappe
- 50: O-Ring
- 52: O-Ring
- 54: Konus / Einsatz
- 56: Konus / Einsatz

## Patentansprüche

1. Austragsvorrichtung (1) für Zementkartuschen für Polymethylmethacrylat-Knochenzemente aufweisend mindestens einen manuell zu betätigenden Kipphebel (4), einen Adapter (44) für eine Zementkartusche und eine in Richtung des Adapters (44) vortreibbare Klemmstange (2),
wobei an der Klemmstange (2) anliegend zumindest ein Vortriebskörper (24) angeordnet ist, der in axialer Richtung bezüglich der Achse der Klemmstange (2) verschiebbar ist,
wobei der zumindest eine an der Klemmstange (2) anliegende Vortriebskörper (24) mit der Klemmstange (2) zumindest eine Kavität bildet, wobei die Kavität eine in der Vortriebsrichtung der Klemmstange (2) geneigte Fläche oder Kante aufweist, so dass sich die Kavität im Bereich dieser Fläche oder Kante in Richtung des Adapters (44) weitet,
wobei mindestens ein kugel- oder walzenförmiger Klemmkörper (26) in der Kavität des zumindest einen Vortriebskörpers (24) angeordnet ist, wobei der mindestens eine Klemmkörper (26) des zumindest einen Vortriebskörpers (24) an mindestens einem Punkt punktuell oder entlang einer Linie die Klemmstange (2) berührt,
wobei ein elastisches Element (28, 52) den mindestens einen Klemmkörper (26) des zumindest einen Vortriebskörpers (24) aus Richtung des Adapters (44) an die geneigte Fläche oder Kante der Kavität des zumindest einen Vortriebskörpers (24) drückt,
wobei der Kipphebel (4) drehbar gegen die Klemmstange (2) gelagert ist, so dass bei Betätigung des Kipphebels (4) ein Ende (8) des Kipphebels (4) auf den zumindest einen axial verschiebbaren Vortriebskörper (24) in Vortriebsrichtung der Klemmstange (2) drückt,
wobei ein Federelement (40) entgegengesetzt zur Vortriebsrichtung der Klemmstange (2) auf den zumindest einen Vortriebskörper (24) drückt, zumindest ein Sicherungselement (34) an der Klemmstange (2) anliegt, gegen das der Vortriebskörper (24) beweglich gelagert ist, wobei das zumindest eine an der Klemmstange (2) anliegende Sicherungselement (34) mit der Klemmstange (2) zumindest eine Kavität bildet, wobei die Kavität eine zur Vortriebsrichtung der Klemmstange (2) geneigte Fläche oder Kante aufweist, so dass sich die Kavität im Bereich dieser Fläche oder Kante in Richtung des Adapters (44) weitet,
wobei mindestens ein kugel- oder walzenförmiger Klemmkörper (36) in der Kavität des zumindest einen Sicherungselements (34) angeordnet ist, wobei der mindestens eine Klemmkörper (36) des zumindest einen Sicherungselements (34) an mindestens einem Punkt punktuell oder entlang einer Linie die Klemmstange (2) berührt,
wobei ein elastisches Element (38, 50) den mindestens einen Klemmkörper (36) des zumindest einen Sicherungselements (34) aus Richtung des Adapters (44) an die geneigte Fläche oder Kante der Kavität des zumindest einen Sicherungselements (34) drückt.

2. Austragsvorrichtung (1) nach Anspruch 1, **dadurch gekennzeichnet, dass** die Austragsvorrichtung (1) ein Gehäuse (6) mit einem Handgriff (22) aufweist, wobei vorzugsweise das zumindest eine Sicherungselement (34) fest mit dem Gehäuse (6) verbunden ist und der zumindest eine Vortriebskörper (24) beweglich gegen das Gehäuse (6) gelagert ist.

3. Austragsvorrichtung (1) nach Anspruch 1 oder 2, **dadurch gekennzeichnet, dass**
der zumindest eine Vortriebskörper (24) die Klemmstange (2) zumindest bereichsweise umschließt, bevorzugt zu mindestens 75% umschließt, besonders bevorzugt vollständig umschließt.

4. Austragsvorrichtung (1) nach Anspruch 3, **dadurch gekennzeichnet, dass** der zumindest eine Vortriebskörper (24) eine Durchführung aufweist, die bevorzugt als Röhre ausgebildet ist.

5. Austragsvorrichtung (1) nach Anspruch 1 oder 2, **dadurch gekennzeichnet, dass**
mehrere axial verschiebbare Vortriebskörper (24) aus unterschiedlichen Richtungen an der Klemmstange (2) anliegen.

6. Austragsvorrichtung (1) nach einem der vorangehenden Ansprüche, **dadurch gekennzeichnet, dass**
die geneigte Fläche derart geformt ist, dass der zumindest eine Vortriebskörper (24) und das zumindest eine Sicherungselement (34) mindestens eine im Längsschnitt in Richtung des Adapters (44) geneigte Kavität aufweist, bevorzugt eine keilförmige Kavität aufweist, wobei die Keilform in Richtung des Adapters (44) geöffnet ist.

7. Austragsvorrichtung (1) nach einem der vorangehenden Ansprüche, **dadurch gekennzeichnet, dass**
der zumindest eine kugel- oder walzenförmige Klemmkörper (26, 36) um zumindest eine Achse drehbar in der Kavität des zumindest einen Vortriebskörpers (24) und/oder des zumindest einen Sicherungselements (34) angeordnet oder gelagert ist.

8. Austragsvorrichtung (1) nach einem der vorangehenden Ansprüche, **dadurch gekennzeichnet, dass**
die Kavität, die durch den zumindest einen Vortriebskörper (24) und die Klemmstange (2) gebildet ist, und/oder die Kavität, die durch das zumindest eine Sicherungselement (34) und die Klemmstange (2) gebildet ist, als Trichter ausgebildet ist oder sind, wobei der Trichter die Klemmstange (2) umschließt.

9. Austragsvorrichtung (1) nach einem der vorangehenden Ansprüche, **dadurch gekennzeichnet, dass**
die Klemmkörper (26, 36) aus Keramik oder Metall gebildet sind und besonders bevorzugt aus Stahl und/oder Wolfram.

10. Austragsvorrichtung (1) nach einem der vorangehenden Ansprüche, **dadurch gekennzeichnet, dass**
die geneigte Fläche oder Kante der Kavität des zumindest einen Vortriebskörpers (24) und/oder des zumindest einen Sicherungselements (34) aus Metall oder aus einem Metall-Kunststoffverbund besteht.

11. Austragsvorrichtung (1) nach einem der vorangehenden Ansprüche, **dadurch gekennzeichnet, dass**
das zumindest eine Sicherungselement (34) auf der Klemmstange (2) vor oder auch hinter dem zumindest einen Vortriebskörper (24) in Bezug zur Vortriebsrichtung der Klemmstange (2) angeordnet ist.

12. Austragsvorrichtung (1) nach einem der vorangehenden Ansprüche, **dadurch gekennzeichnet, dass**
die Klemmkörper (36) und die Klemmstange (2) eine Härte von mindestens 50 HRC haben.

13. Austragsvorrichtung (1) nach einem der vorangehenden Ansprüche, **dadurch gekennzeichnet, dass**
die elastischen Elemente (28, 52, 38, 50), die den zumindest einen Klemmkörper (26) des zumindest einen Vortriebskörpers (24) und den zumindest einen Klemmkörper (36) des zumindest einen Sicherungselements (34) an die geneigten Flächen und/oder Kanten der Kavitäten des zumindest einen Vortriebskörpers (24) und des zumindest einen Sicherungselements (34) drücken, elastisch Körper sind, die vorzugsweise die Kavitäten in Richtung des Adapters (44) verschließen.

14. Austragsvorrichtung (1) nach einem der vorangehenden Ansprüche, **dadurch gekennzeichnet, dass**
der Kraftschluss des zumindest einen Klemmkörpers (26) des zumindest einen Vortriebskörpers (24) mit der Klemmstange (2) und der Kraftschluss des zumindest einen Klemmkörpers (36) des zumindest einen Sicherungselements (34) mit der Klemmstange (2) nicht gleichzeitig lösbar sind, so dass die Klemmstange (2) mit der Austragsvorrichtung nur unidirektional vorzutreiben ist.

15. Verfahren zum Vortreiben einer Klemmstange (2) mit einem Kipphebel (4) bei dem durch manuelle Krafteinwirkung auf den Kipphebel (4) zumindest ein an der Klemmstange (2) anliegender Vortriebskörper (24) in einer ersten axialen Richtung der Klemmstange (2) bewegt wird,
wobei zumindest ein Klemmkörper (26) durch die Kraft der Bewegung gegen eine geneigte Fläche oder Kante der Kavität an dem zumindest einen Vortriebskörper (24) und die Klemmstange (2) gedrückt wird, wobei der Klemmkörper (26) in einer Kavität des zumindest einen Vortriebskörpers (24) angeordnet ist, wobei die Kavität durch den zumindest einen Vortriebskörper (24) und die Klemmstange (2) gebildet wird, und wobei der Klemmkörper (26) die Klemmstange (2) an wenigstens einem Punkt oder entlang einer Linie berührt, und wobei der zumindest eine Klemmkörper (26) und der zumindest eine Vortriebskörper (24) durch den Druck derart kraftschlüssig mit der Klemmstange (2) verbunden sind, dass die Klemmstange (2) mit dem Vortriebskörper (24) bewegt wird,
wobei sobald die manuelle Krafteinwirkung auf den Kipphebel (4) reduziert wird oder beendet wird der zumindest Vortriebskörper (24) durch ein Federelement (40) in die entgegengesetzte axiale zweite axiale Richtung gedrückt wird, wobei der Kipphebel (4) wieder in die Ausgangsstellung gekippt wird und wobei sich durch die umgekehrte Krafteinwirkung der zumindest eine Klemmkörper (26) von der geneigten Fläche oder Kante des zumindest einen Vortriebskörpers (24) in der Kavität löst und dadurch über die Klemmstange (2) gleitet und/oder abrollt,
wobei eine umgekehrte Bewegung der Klemmstange (2) in die zweite axiale Richtung durch zumindest ein an der Klemmstange (2) anliegendes Sicherungselement (34) blockiert wird, wobei zumindest ein Klemmkörper (36) des Sicherungselements (34) durch die umgekehrte Bewegung der Klemmstange (2) gegen eine geneigte Fläche oder Kante der Kavität an dem zumindest einen Sicherungselement (34) und die Klemmstange (2) gedrückt wird, wobei der Klemmkörper (36) in einer Kavität des zumindest einen Sicherungselements (34) angeordnet ist, die durch das zumindest eine Sicherungselement (34) und die Klemmstange (2) gebildet wird, und wobei der Klemmkörper (36) die Klemmstange (2) an wenigstens einem Punkt oder entlang einer Linie berührt, und der zumindest eine Klemmkörper (36) und das zumindest eine Sicherungselement (34) durch den Druck auf den Klemmkörper (36) derart kraftschlüssig mit der Klemmstange (2) verbunden sind, dass die Klemmstange (2) sich nicht gegen das zumindest eine Sicherungselement (34) bewegt.

16. Verfahren nach Anspruch 15, **dadurch gekennzeichnet, dass** sich bei einer Bewegung der Klemmstange (2) in die erste axiale Richtung der zumindest eine Klemmkörper (36) des zumindest einen Sicherungselements (34) von der geneigten Fläche oder Kante der Kavität des zumindest einen Sicherungselements (34) löst und die Klemmstange (2) dadurch gegen das zumindest eine Sicherungselement (34) bewegt wird.

17. Verfahren nach Anspruch 15 oder 16, **dadurch gekennzeichnet, dass** der zumindest eine Klemmkörper (26) des zumindest einen Vortriebskörpers (24) und der zumindest eine Klemmkörper (36) des zumindest einen Sicherungselements (34) durch Kugeln und/oder Walzen gebildet werden, die auf der Klemmstange (2) zumindest geringfügig abrollen.

18. Verfahren nach einem der Ansprüche 15 bis 17, **dadurch gekennzeichnet, dass**
die geneigte Fläche oder Kante der Kavität des zumindest einen Vortriebskörpers (24) und/oder des zumindest einen Sicherungselements (34) im Querschnitt keilförmig ist.

19. Verfahren nach einem der Ansprüche 15 bis 18, **dadurch gekennzeichnet, dass**
beim Kippen des Kipphebels (4) durch manuelle Krafteinwirkung das Federelement (40) komprimiert wird.

20. Verfahren nach einem der Ansprüche 15 bis 19, **dadurch gekennzeichnet, dass**
ein elastisches Element (38, 50) den mindestens einen Klemmkörper (36) des zumindest einen Sicherungselements (34) aus Richtung des Adapters (44) an die geneigte Fläche oder Kante der Kavität des zumindest einen Sicherungselements (34) drückt.

21. Verfahren nach einem der Ansprüche 15 bis 20, **dadurch gekennzeichnet, dass**
die Bewegung mehrfach wiederholt wird und dabei die Klemmstange (2) schubweise vorgetrieben wird und dabei schubweise ein Knochenzement aus einer Kartusche ausgetrieben wird, die zuvor über einen Adapter (44) mit einer Austragsvorrichtung (1) verbunden wurde, wobei die Austragsvorrichtung (1) den Adapter (44), den Kipphebel (4), die Klemmstange (2), den zumindest einen Vortriebskörper (24) und das zumindest eine Sicherungselement (34) umfasst.

22. Verfahren nach einem der Ansprüche 15 bis 21, **dadurch gekennzeichnet, dass**
das Verfahren durch Verwendung einer Austragsvorrichtung (1) nach einem der Ansprüche 1 bis 13 durchgeführt wird.
